# EUROPEAN PATENT SPECIFICATION

(11) **EP 1 502 561 B1**
(45) Date of publication and mention of the grant of the patent: **18.10.2006**
(21) Application number: 04254421.3
(22) Date of filing: 23.07.2004
(51) Int. Cl.: A61F 2/16

(54) **Intraocular lens**
Intraocularlinse
Lentille intraoculaire

(30) Priority: 31.07.2003 US 633209
(43) Date of publication of application: 02.02.2005
(73) Proprietor: Rayner Intraocular Lenses Limited, Chesham, Bucks HP5 1DJ (GB)
(72) Inventor: Toop, Peter, Rayner Intraocular Lenses Ltd., Hove, East Sussex BN3 7AN (GB)
(74) Representative: Perry, Robert Edward

(56) References cited:
- EP-A- 0 962 196
- WO-A-20/04010895
- US-A- 5 674 283
- US-B1- 6 179 870
- US-B1- 6 190 410

## Description

This invention relates to an intraocular lens comprising a toric optic.

### Background of the Invention

A typical intraocular lens comprises an optic and two opposed haptics. Conventional intraocular lenses are often prone to "haptic failure", wherein excessive capsular contraction causes the haptics to buckle or twist, resulting in their dislocation. The haptics should maintain the optic centrally in the capsular sac and should not be so springy as to damage the sac after insertion.

EP-A-0962196 describes an intraocular lens wherein the haptics are shaped such that, in a first stage of compression, the proximal part of the haptic can be fully compressed; and in a second stage, the distal part of the haptic can be compressed, to provide a lens that is essentially resistant to haptic failure.

US-B1-6179870 discloses an intraocular lens according to the preamble of claim 1.

Astigmatism is a visual defect that can arise from an irregularity in the shape of the cornea. An astigmatic cornea is defined by spherical and cylindrical axes, the vertical meridian providing a different optical power to that of the horizontal meridian. The unequal curvature of the cornea causes light entering the eye to focus at different points, thus distorting vision. Astigmatism is often coupled with other visual impairments such as myopia and hypermetropia.

Astigmatism can be corrected by using a toric lens. A toric lens is designed to match the unequal curvatures of an astigmatic cornea. One of the requirements of a toric lens is that the optic must remain correctly aligned along the spherical and cylindrical axes. Thus, whereas spherical lenses may rotate freely, toric lenses require a means with which to inhibit rotation of the lens. In contact lenses, this is often accomplished by slightly weighting the lens, e.g. by making one or more sections of the lens periphery thicker (or thinner) than other sections.

### Summary of the Invention

According to the present invention, an intraocular lens comprises a toric optic and one or more haptics, wherein the thickness of a region of the distal part of the or each haptic is greater than the rest of the haptic, such that rotation of the lens in the optic plane is inhibited in use. The thicker region of the distal part is preferably a peripheral region.

Preferably, the or each haptic is compressible, in the plane of the lens. More preferably, the or each haptic is curved, and shaped such that, in a first stage of compression, the proximal part of the haptic can be fully compressed and, in a second stage, the distal part of the haptic can be compressed.

The or each haptic may include an aperture, of which opposed points are brought into contact in a first stage of compression. The or each stage of compression may be essentially continuous, full compression being reached gradually from the proximal end towards the distal end of the haptic. A lens of the invention preferably comprises two or more haptics, compression of the haptics preferably providing an essentially elliptical form of the lens.

In use, the thicker distal region(s) of the haptic(s), nestles into the wall of the capsular sac, inhibiting rotation of the lens. This allows the toric optic to remain correctly aligned along the spherical and cylindrical axes. A compressible haptic may further stabilise the lens during use.

### Brief Description of the Drawings

In the accompanying drawings (which are given by way of example only):
Figs. 1A and 1B are respectively plan and side views of an intraocular lens embodying the present invention. Fig. 1C depicts the same lens in a capsular sac.
Fig. 2 is a comparative diagram showing the effect of capsule sac size on a lens embodying the present invention and two conventional toric lenses.

### Description of the Preferred Embodiments

Embodiments of the invention will now be described by way of example only, with reference to the accompanying drawings.

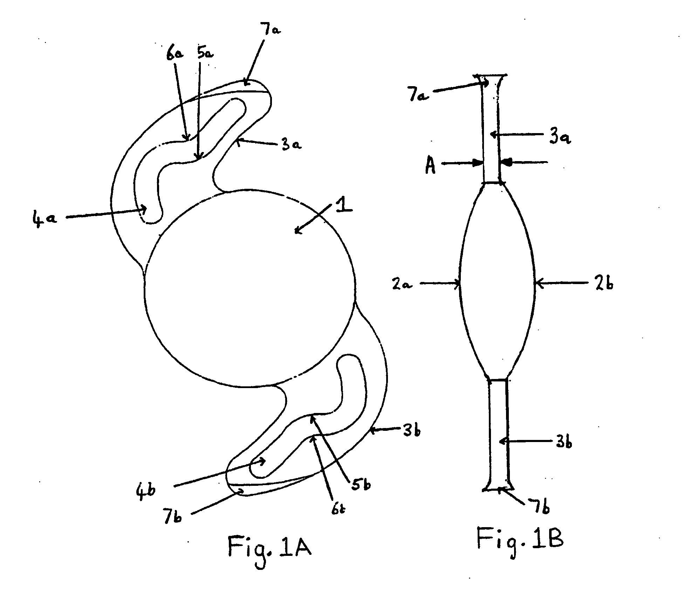

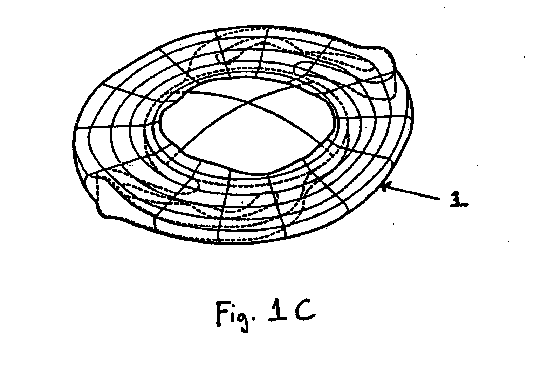

Figs. 1A and 1B show an intraocular lens having a toric optic 1, comprising convex faces 2a and 2b. The lens comprises haptics 3a and 3b, each joined to the optic. Each haptic comprises an aperture, 4a and 4b. Opposed points of each haptic 5a and 6a, and 5b and 6b, are shown.

These features are such that initial compression of the haptic leads to abutment of opposite walls of the aperture, bringing the opposed points into contact, thereby defining a proximal part that is fully compressed and a distal part that can undergo further compression. Such further compression brings the distal end of each haptic substantially into contact with the periphery of the optic, to give an essentially elliptical shape, in plan. These features allow restricted rotation of the lens.

Figs. 1A and 1B also show the thicker peripheral regions 7a and 7b of the distal part of each haptic; haptic thickness is denoted by the dimension A shown in Fig. 1B. These thicker regions inhibit rotation of the lens when in use, thus stabilising the optic along the spherical and cylindrical axes. Fig. 1C shows a capsular sac 1 surrounding the same lens (shown by dashed lines). The thicker distal regions nestle in the wall of the sac, inhibiting rotation of the lens.

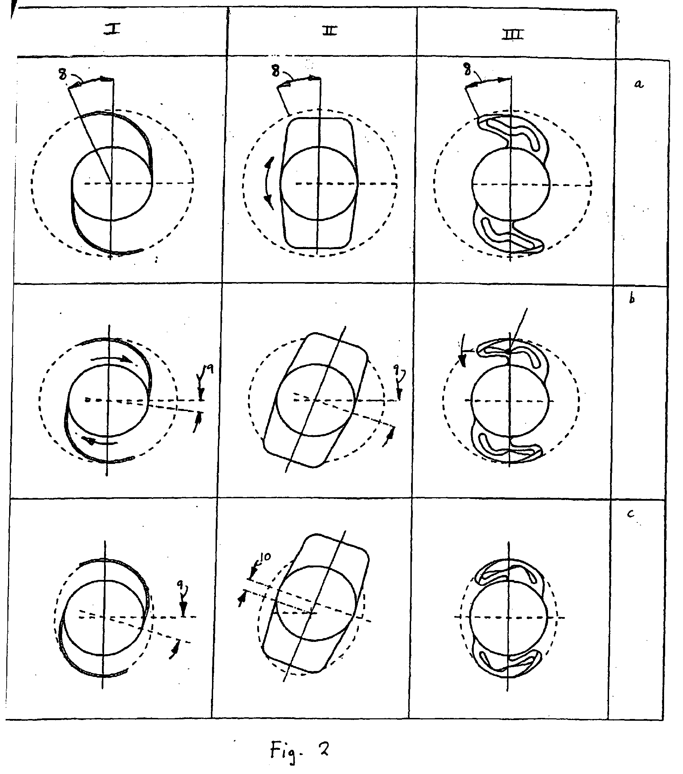

Fig. 2 shows the effect of capsular sac size for a variety of intraocular lenses. Column I depicts a known "C-loop" lens, column II a known "plate-haptic" lens and column III a lens embodying the present invention. Rows a, b and c, correspond to large, average and extremely contracted capsular sacs respectively. The intended angular position, rotational misalignment and translational misalignment are shown by labels 8, 9 and 10 respectively.

In each example of the "C-loop" lens, the optic of the lens can rotate, causing the toric optic to misalign. Rotation and thus misalignment becomes more severe as the capsular size decreases.

In a large capsular sac, the "plate-haptic" lens can rotate to an unpredictable position. When the capsule contracts to an average size, the haptic can fixate, but the lens often becomes rotationally misaligned. In the case of the severely contracted capsule, the lens may undergo translation causing the haptics to rupture the wall of the sac.

In each example of a lens of the invention, the thicker distal regions of the haptics nestle into the wall of the sac, inhibiting rotation. For the large sac, there is little haptic compression but the thicker distal regions still restrict rotation. For the average sac, the semi-compressed haptics inhibit rotation, with the thicker regions further stabilising the lens. Rotation is similarly inhibited in the contracted sac, except that the haptics are now fully compressed.

## Claims

1. An intraocular lens comprising an optic (1) and one or more haptics (3a, 3b), wherein the thickness of a region of the distal part of the or each haptic is greater than the rest of the haptic, **characterised in that** the optic (1) is toric and rotation of the lens in the optic plane is inhibited.

2. A lens according to claim 1, wherein the thickness of the or each haptic is greatest at the periphery.

3. A lens according to claim 1 or claim 2, wherein the or each haptic is compressible, in the plane of the lens.

4. A lens according to claim 3, wherein the or each haptic is curved, and shaped such that, in a first stage of compression, the proximal part of the haptic can be fully compressed and, in a second stage, the distal part of the haptic can be compressed.

5. A lens according to claim 4, wherein the or each haptic includes an aperture (4a, 4b) of which opposed points (5a, 6a; 5b, 6b) are brought into contact, in the first stage of compression.

6. A lens according to claim 4 or claim 5, wherein the or each stage of compression is essentially continuous, full compression being reached gradually from the proximal end towards the distal end of the haptic.

7. A lens according to any of claims 4 to 6, which comprises two or more haptics, wherein the haptics are compressed to provide an essentially elliptical form of the lens.

## Patentansprüche

1. Intraokularlinse mit einer Optik (1) und einer oder mehrerer Haptiken (3a, 3b), wobei die Dicke einer Region des distalen Teils der oder jeder Haptik größer als der Rest der Haptik ist, **dadurch gekennzeichnet, dass** die Optik (1) torisch ist und die Rotation der Linse in der Optikebene gehemmt ist.

2. Linse gemäß Anspruch 1, bei der die Dicke der oder jeder Haptik an der Peripherie am größten ist.

3. Linse gemäß Anspruch 1 oder Anspruch 2, bei der die oder jede Haptik in der Ebene der Linse komprimierbar ist.

4. Linse gemäß Anspruch 3, bei der die oder jede Haptik gekrümmt und derart geformt ist, dass in einer ersten Kompressionsstufe der proximale Teil der Haptik vollständig komprimiert werden kann und in einer zweiten Stufe der distale Teil der Haptik komprimiert werden kann.

5. Linse gemäß Anspruch 4, bei der die oder jede Haptik eine Apertur (4a, 4b) aufweist, von der gegenüberliegende Punkte (5a, 6a; 5b, 6b) in der ersten Kompressionsstufe in Kontakt gebracht werden.

6. Linse gemäß Anspruch 4 oder Anspruch 5, bei der die oder jede Kompressionsstufe im Wesentlichen kontinuierlich ist, wobei eine vollständige Kompression allmählich von dem proximalen Ende zu dem distalen Ende der Haptik hin erreicht wird.

7. Linse gemäß einem der Ansprüche 4 bis 6, die zwei oder mehr Haptiken umfasst, wobei die Haptiken komprimiert werden, um eine im Wesentlichen elliptische Form der Linse bereitzustellen.

## Revendications

1. Lentille intraoculaire comprenant une optique (1) et un ou plusieurs porteurs (3a, 3b), dans laquelle l'épaisseur d'une région de la partie distale du ou de chaque porteur est supérieure au reste du porteur, **caractérisée en ce que** l'optique (1) est torique et la rotation de la lentille dans le plan optique est empêchée.

2. Lentille selon la revendication 1, dans laquelle l'épaisseur du ou de chaque porteur est la plus importante à la périphérie.

3. Lentille selon la revendication 1 ou la revendication 2, dans laquelle le ou chaque porteur est compressible, dans le plan de la lentille.

4. Lentille selon la revendication 3, dans laquelle le ou chaque porteur est courbe et présente une forme telle que, au cours d'une première étape de compression, la partie proximale du porteur peut être complètement compressée et, au cours d'une seconde étape, la partie distale du porteur peut être compressée.

5. Lentille selon la revendication 4, dans laquelle le ou chaque porteur comporte une ouverture (4a, 4b) dont les points opposés (5a, 6a ; 5b, 6b) sont amenés en contact, au cours de la première étape de compression.

6. Lentille selon la revendication 4 ou la revendication 5, dans laquelle la ou chaque étape de compression est essentiellement continue, la compression totale étant atteinte de façon progressive de l'extrémité proximale vers l'extrémité distale du porteur.

7. Lentille selon l'une quelconque des revendications 4 à 6, laquelle comprend deux ou plusieurs porteurs, dans laquelle les porteurs sont comprimés afin de conférer une forme pratiquement elliptique de la lentille.
